# EUROPÄISCHE PATENTSCHRIFT

(11) **EP 1 759 722 B1**
(45) Veröffentlichungstag und Bekanntmachung des Hinweises auf die Patenterteilung: **11.11.2009**
(21) Anmeldenummer: 05019053.7
(22) Anmeldetag: 01.09.2005
(51) Int. Cl.: A61L 27/30, C23C 14/00

(54) **Beschichtung von Teilen aus Titan oder einer Titan-Legierung zur Verhinderung von Kaltverschweissung**
Coating of titan or titan alloy parts for avoiding cold welding
Revêtement d'éléments en titane ou en alliage de titane pour empêcher la soudure à froid

(43) Veröffentlichungstag der Anmeldung: 07.03.2007
(73) Patentinhaber: Straumann Holding AG, 4002 Basel (CH)
(72) Erfinder: Grohmann, Fred-Rainer, 8902 Urdorf/LH (CH); de Wild, Michael, 4104 Oberwil (CH); Memmolo, Marcello, 4450 Sissach (CH)
(74) Vertreter: Modiano, Micaela Nadia

(56) Entgegenhaltungen:
- EP-A- 0 295 397
- EP-A- 0 401 488
- EP-A- 0 418 905
- EP-A- 1 136 585
- FR-A- 2 576 793
- LIU X ET AL: "Surface modification of titanium, titanium alloys, and related materials for biomedical applications" MATERIALS SCIENCE AND ENGINEERING R: REPORTS, ELSEVIER SEQUOIA S.A., LAUSANNE, CH, Bd. 47, Nr. 3-4, 24. Dezember 2004 (2004-12-24), Seiten 49-121, XP004722113 ISSN: 0927-796X
- THULL R: "Development And Electrochemical Evaluation Of Two PVD-hard Coatings For Implants Made Of Titanium" ENGINEERING IN MEDICINE AND BIOLOGY SOCIETY, 1991. VOL.13: 1991., PROCEEDINGS OF THE ANNUAL INTERNATIONAL CONFERENCE OF THE IEEE ORLANDO, FL, USA 31 OCT.-3 NOV. 1991, NEW YORK, NY, USA,IEEE, US, 31. Oktober 1991 (1991-10-31), Seiten 1477-1478, XP010102162 ISBN: 0-7803-0216-8

## Beschreibung

Die vorliegende Erfindung betrifft an Verfahren zur Beschichtung von Elementen aus Titan oder Titan-Legierung im Bereich der Medizin- oder Medizinaltechnik gemäß den Ansprüchen, wie z. B. Knochenschrauben, Implantate, Dentalimplantate, Übertragungsteile, (Knochen-)Platten, Implantierhilfen oder Werkzeuge wie Pinzetten, Bohrer usw. aus Titan oder Titan-Legierung, die mit anderen Elementen aus Titan oder Titan-Legierungen in Berührung kommen.

Schraubenimplantate werden in eine im Knochen bzw. Kieferknochen vorbereitete Bohrung eingedreht oder besitzen ein selbstschneidendes Aussengewinde, das sich beim Eindrehen in ein präpariertes Sackloch das Innengewinde selbst erzeugt. Zylinderimplantate hingegen sind äusserlich gewindelos und werden in eine im Knochen vorbereitetes Sackloch eingedrückt. Die vorliegende Erfindung bezieht sich vorrangig auf enossale Dentalimplantate in Schraubenform.

Übertragungsteile sind dazu da, Implantate in der Verpackung während Transport und Lagerung stabil zu halten, zum Anfassen und Herausnehmen und zum Eindrehen des Implantates in das vorgebohrte Loch im Knochen. Für das Handling der vorgenannten Implantate sind verschiedene Adapter bzw. Übertragungsteile bekannt. Für die Aufbewahrung solcher Implantate sind vielfältige gestaltete Ampullen auf dem Markt, die der sterilen Aufbewahrung dienen.

Aus der WO-A1-98 55039 ist ein rotationssymmetrisches Übertragungsteil für ein in Knochen einzusetzendes Implantat, insbesondere ein Dentalimplantat und eine Innenampulle oder Ampulle für die Aufbewahrung des Implantats bekannt, worin das Übertragungsteil mittels eines Schraubenschafts schraubend mit dem Implantat im Eingriff bringbar ist. Die Einheit, bestehend aus dem Übertragungsteil und dem darauf verschraubten Implantat, kann in eine Innenampulle zur Aufbewahrung und zum Transport des Implantats eingebracht werden, die eine Fixierpartie aufweist, in welche das Übertragungsteil direkt oder indirekt montierbar ist und die eine seitliche, der Länge der Einheit entsprechende Aussparung hat, durch die das Implantat mit dem Übertragungsteil entfernbar ist. Das Übertragungsteil aus dem Stand der Technik weist an dem, dem Schraubenschaft entgegengesetzten Ende, einen Fortsatz mit einem Aussenvielkant (insbesondere einem Achtkant) auf, auf dem ein Werkzeug, z. B. ein Eindrehwerkzeug, aufsteckbar ist. Ein Sicherungselement, vorzugsweise ein O-Ring, ist in eine Radialnut unterhalb des Fortsatzes vorgesehen. Darüber hinaus wird gemäss der WO-A1-98 55039 eine Aussenkapsel mit einem darauf aufschraubbaren Verschlussdeckel derart ausgebildet, dass das Übertragungsteil mit dem Implantat axial zwischen dem Boden der Aussenkapsel und dem Verschlussdeckel arretierbar ist. Der Gesamtinhalt der WO-A1-98 55039 wird durch die Bezugnahme hierin eingeschlossen.

US-B1-6 247 932 beschreibt einen Behälter für ein dentales Implantat, worin das Übertragungsteil einrastend mit dem Implantat verbunden ist. Der bekannte Behälter ist analog zur Aussenkapsel der WO-A1-98 55039 ausgebildet, um das Übertragungsteil mit dem aufgeschnappten Implantat aufzunehmen und zu sichern. Zusätzlich sieht US-B1-6 247932 Mittel zur Drehsicherung des Implantats im Bezug zum Übertragungsteil bzw. Mittel zur Kraftübertragung vom Übertragungsteil auf das Implantat vor.

Ein Übertragungsteil mit einem Schnappmechanismus ist aus der US-B1-6 206 696 bekannt, wobei die Verbindung mit dem Implantat durch ein Einschnappen in eine Einheilschraube erfolgt. Ähnliche Übertragungsteile sind aus der WO-A1-00 2496 und der WO-A1-01 50978 bekannt, die mit Hilfe eines Einschnappvorgangs mit einer Einheilschraube, die am Implantat befestigt ist, verbindbar sind.

Übertragungsteile werden entweder mit dem Implantat verschraubt oder in bzw. auf das Implantat montiert. Geschraubte Übertragungsteile haben dabei den Nachteil, dass sie vom Implantatkopf gelöst werden, indem das Übertragungsteil an einer geeigneten Stelle mit einem Konterschlüssel - bei gleichzeitiger Fixierung des frisch gesetzten Implantats mit einem zweiten Schlüssel - gefasst wird und so vorsichtig gelöst wird. Insbesondere bei Dentalimplantaten - im engen Raum eines Patientenmundes mit vorhandenen Nachbarzähnen, insbesondere im anterioren Bereich - erfordern diese Arbeitsabläufe mit zwei Instrumenten eine hohe Fertigkeit des operierenden Chirurgen, sind daher stets diffizil und zeitaufwendig. Bei wechselnden Positionen der eingesetzten Implantate, insbesondere im Ober- und Unterkiefer, kann es zudem problematisch sein, auf Anhieb die lösende Richtung beim Abnehmen des Adapters zu erkennen. Neuere Lösungen weisen daher eine geschnappte Verbindung auf, die einfacher lösbar ist.

In bestimmten Fällen ist es vorteilhaft das Implantat mit vormontiertem Übertragungsteil nicht wie üblicherweise trocken zu lagern sondern in einer Flüssigkeit wie z. B. Wasser oder einer Salzlösung. Dies ist z. B. nötig, wenn die Oberfläche des Implantates besonders veredelt ist (durch Beschichtung mit Peptiden, Lipiden oder Proteinen), so dass eine Lagerung in einer geeigneten Flüssigkeit notwendig ist. Dies gilt bspw. auch für eine hydrophile Oberfläche, wie sie in der EP 1150620 beschrieben wird. Die Hydrophilie, Ladung, chemische Aktivität und chemische Sauberkeit der Oberfläche sind vor atmosphärischen Verunreinigungen wie Kohlenstoffdioxid oder Kohlenwasserstoffen geschützt, wenn das Implantat in reinem Wasser, welches gegebenenfalls mit ausgewählten Zusatzstoffen versehen ist, gelagert wird.

Nach der Lagerung von Implantaten mit aufmontierten Übertragungsteilen, insbesondere nach der Lagerung in einer wässrigen Umgebung (z. B. in einer isotonischen Kochsalzlösung), wurde überraschenderweise festgestellt, dass sich die beiden Elemente nicht mehr voneinander lösen liessen. Es kommt augenscheinlich in diesem Fall zu einer "Kaltverschweissung" der beiden Elemente, die jeweils aus Titan bzw. einer Titan-Legierung bestehen und eigentlich durch die sich automatisch auf der Oberfläche des Titans ausbildende Titanoxid-Schicht geschützt sein sollten. Bei der Lagerung der montierten Elemente läuft wahrscheinlich eine instantane Neu-Oxidierung - z. B. im Fall eines Kratzers / Verletzung der schützenden Titan-Oxidschicht - oder Weiterbildung der Oxidschicht auf der Oberfläche des Titans ab, durch die die beiden Elemente dann fest miteinander "verschweisst" werden und anschliessend nicht mehr voneinander getrennt werden können. Dieser Vorgang ist bei einer Lagerung in Wasser oder wässrigen Lösungen massiv beschleunigt.

Der Effekt der Kaltverschweissung ist für Implantate aus Titan bereits bekannt. So nutzt der Implantathersteller Zimmer die Kaltverschweissung sogar positiv - alle AdVent Implantate verfügen über eine Innensechskantverbindung mit Friktionsanpassung. Dabei gehen Innensechskante mit über Friktion eingepassten Aufbauten beabsichtigt eine Kaltverschweissung mit dem Implantat ein, wodurch Mikrobewegungen grundlegend beseitig werden und die Verbindung spaltfrei und bakteriendicht wird (Produkt Katalog Januar 2004 der Zimmer Dental GmbH). In einem solchen Fall ist der Effekt der Kaltverschweissung vorteilhaft und erwünscht. Wenn jedoch die titanhaltigen Elemente nach der Implantation wieder getrennt werden sollen, ist die Kaltverschweissung sehr problematisch. Wenn sich z. B. die Knochenschrauben mit der Osteosyntheseplatte kaltverschweissen, können diese bei der Entfernung des Osteosynthesematerials auch mit maximaler Kraftanstrengung nicht mehr aus der Platte gelöst werden, und es sind entsprechende Ersatztechniken meist brachialer Art notwendig. Auch bei der Nutzung von Übertragungsteilen für Implantate muss gewährleistet sein, dass diese nach der Implantation problemlos voneinander gelöst werden können.

Als naheliegende Lösung bieten sich Oberflächenbehandlungen oder Beschichtungen an, so dass eine Schutz- oder Trennschicht zwischen den beiden Titanelemente (aus reinem Titan oder einer Titan-Legierung) vorhanden ist. Diese verhindern das Kaltverschweissen der Elemente und fördert gleichzeitig die tribologischen Eigenschaften wie Gleitverhalten bzw. trockenere Schmierung. Dabei sollte die Schutz- oder Trennschicht fest angebunden sein, inert bei der Lagerung in Flüssigkeiten wie Wasser oder wässrigen Salzlösungen sowie biokompatibel. Es ist wichtig, dass die Schicht inert ist, d.h. unlöslich in der Flüssigkeit ist, da ansonsten die Oberfläche des Implantates kontaminiert oder verändert werden kann. Mögliche Oberflächenbehandlungen sind z. B. Trovalisieren, mechanisches Polieren, Elektropolieren, Anodisieren oder das Strahlen mit Glaskugeln, Stahlkugeln, Titanoxid oder Laser. Auch Beschichtungen mit Gold, Diamant (ADLC - amorph diamond-like carbon) oder Metallnitriden wie Zirkoniumnitrid, Titaniumnitrid, Chromnitrid, Wolframnitrid oder Titan-Aluminium-Nitrid sind denkbar. Diese Möglichkeiten wurden ausgetestet, brachten aber keine Verbesserung des "Verschweissen" und wurden daher verworfen.

Aus der EP 1 136 585 A1 ist ein PVD-Verfahren zur Beschichtung von Elementen gemäß Oberbegriff des Anspruchs 1 bekannt.

Aus der EP 0 401 488 A1 ist ein Verfahren zur Beschichtung von einem Substrat aus Titan mit einer Hartstoffschicht, zum Beispiel aus Ti-Niobonitrid, und einer Schicht aus Ti-Nioboxid, mittels PVD bekannt.

Aus der EP 0 295 397 A1 ist ein Verfahren zur Beschichtung eines Substrats aus einer Titan-Legierung bekannt, und zwar mit einer Schicht enthaltend Ti, N, C und O in einer Atmosphäre, bestehend aus Stickstoff und Acetylen.

Es ist daher die Aufgabe der vorliegenden Erfindung, eine Beschichtung vorzuschlagen, die auf einem Element aus Titan oder Titanlegierung, insbesondere einem Übertragungsteil, aufgebracht wird, so dass nach Montage auf einem zweiten Element aus Titan oder Titanlegierung, insbesondere einem Implantat, sowie nach der Lagerung der beiden Elemente in montiertem Zustand in einer Flüssigkeit, beide Elemente ohne Probleme, d. h von Hand bzw. ohne weitere Werkzeuge wieder voneinander getrennt werden können bspw. nach der Implantation in einen Knochen. Eine Trennung soll dabei auch nach Kraftaufwendung (z. B. der Eindrehung mit torsionaler Belastung zwischen den beiden Elementen) möglich sein. Diese Beschichtung sollte einen geringeren Abrieb haben und so beschaffen sein, dass sie die Geometrie- und Oberflächenbeschaffenheit sowie die Biokompatibilität der beiden Elemente nicht beeinflusst bzw. verändert.

Erfindungsgemäss wird die gestellte Aufgabe mittels einer Beschichtung nach Anspruch 8 sowie mittels eines Verfahrens zu deren Herstellung nach Anspruch 1 gelöst. Desweiteren betrifft die Erfindung beschichtete Elemente nach Anspruch 6 aus Titan oder Titan-Legierung, die in der Medizintechnik zur Anwendung kommen.

Die Erfindung basiert auf einem PVD-Verfahren zur Beschichtung von Elementen aus Titan oder Titan-Legierung, das mindestens folgende Schritte aufweist:
a) Positionieren der Elemente in einem Rezipienten;
b) Anlegen eines Vakuum;
c) Abscheidung eines ersten Metalls in Stickstoff- und Azetylen-Atmosphäre;
d) Abscheidung eines zweiten Metalls in Stickstoff- und Azetylen-Atmosphäre;
e) Wiederholung der Schritte c) und d) bis eine ausreichende Schichtdicke erreicht ist;
f) Belüftung des Rezipienten;
g) Entnahme der Elemente aus dem Rezipienten;
h) Reinigung der Elemente mit einem geeigneten Reinigungsbad;

Zusätzlich kann nach dem Schritt b) eine Vorbehandlung der Oberfläche durchgeführt wird, insbesondere durch Sputtern mit Edelgasen. Ausserdem kann während Schritt e) das Verhältnis - insbesondere linear - von Stickstoff und / oder Azetylen verändert werden. Erfindungsgemäß beträgt die Schichtdicke mindestens 1 µm, wobei das erste Metall Chrom und das zweite Metall Zirkon ist.

Diese Anmeldung betrifft auch eine Beschichtung hergestellt in dem erfinderischen Verfahren sowie Elemente aus Titan oder Titan-Legierung mit einer Beschichtung hergestellt in dem erfinderischen Verfahren. Insbesondere betrifft dies Implantate, Dentalimplantate, Knochenschrauben oder Übertragungsteile. Desweiteren betrifft die Erfindung eine Kombination von Elementen, bei denen mindestens ein Element zumindest teilweise mit einer erfinderischen Beschichtung.

Die erfindungsgemässe Schicht besteht aus verschiedenen Einzelschichten, die aus mindestens zwei verschiedenen Metallen bestehen. Die Herstellung der Schicht erfolgt mittels Vakuumabscheidung verschiedener Einzelschichten aus mindestens zwei verschiedenen Metallen.

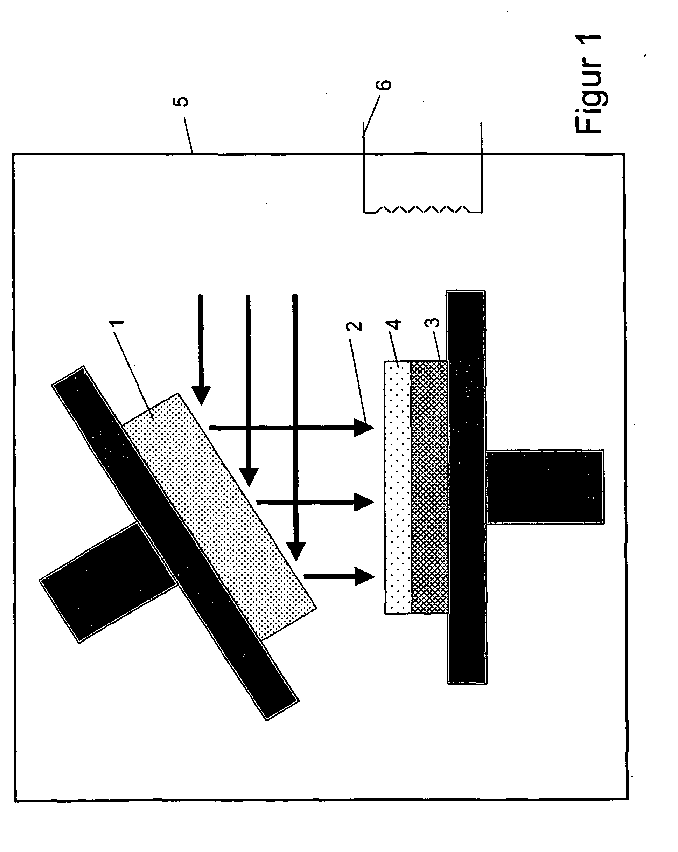

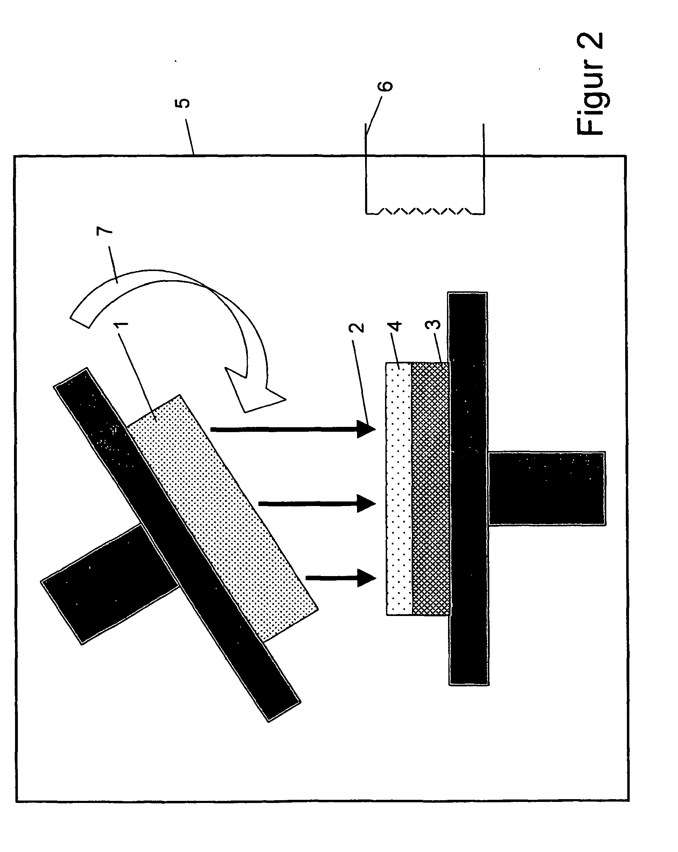

Die Herstellung geschieht mittels sogenanntem PVD-Verfahren (Physical Vapour Deposition). Dieses Verfahren ist in Figur 1 schematisch dargestellt. Dabei wird in einer Vakuumkammer (5) durch Sublimation (oder Sieden) einer festen (oder geschmolzenen) Materialquelle oder Target (1) ein Dampf (2) erzeugt. Die Dampfatome oder -moleküle bewegen sich von der Quelle zum Substrat (3) und kondensieren dort zu einem festen Film (4). Unterschiedliche Aufdampfverfahren unterscheiden sich in der Art wie das Verdampfermaterial aufgeheizt wird: Bogen-Verfahren (auch Arc-Verfahren), Elektronenstrahl-Verfahren, Laser-Verfahren, Molekularstrahl-Epitaxie sowie Thermisches Verfahren. Das Bogenverdampfen (auch Arc-Verdampfen) ist eine spezielle Form des Aufdampfens (siehe Figur 2). Zwischen Anode und dem als Kathode geschalteten Target (Ausgangsmaterial) (1) wird ein Lichtbogen (7) gezündet, der auf der Kathodenoberfläche "wandert". Dort wo der Lichtbogen auf das Target trifft, wird das Material in den gasförmigen Zustand (2) überführt und aufgrund der hohen Ströme und Leistungsdichten zu einem hohen Prozentsatz ionisiert. Das Gas und die Ionen treffen auf das Substrat (3) und führen dort zur Schichtbildung (4).

Bevor die zu beschichtenden Elemente mit einer Beschichtung versehen werden können, müssen sie zuerst absolut fettfrei und mikroskopisch sauber, trocken und frei von jeglichen Oxiden sein. Diese Vorreinigung kann bei der erfindungsgemässen Beschichtung z. B. mit einer 12-Kammer-Ultraschall-Reinigungsanlage geschehen. Anschliessend werden die vorbehandelten Elemente in einen Rezipienten (Beschichtungsanlage) (5) gebracht und an speziellen Halterungen befestigt, die stabil genug für eine Doppel- oder Dreifachrotation sind. Nach Schliessen der Türen wird der Rezipient evakuiert, bis ein Endvakuum von mindestens 1,0 x 10⁻⁶ mbar vorliegt. Um dieses Vakuum zu erreichen, werden z. B. Turbomolekularpumpen verwendet.

Bei der Beschichtung ist es vorteilhaft, wenn die zu beschichteten Elemente bzw. Substrate eine gewisse Temperatur haben, wie beispielsweise ca. 200°C, um so durch epitaktisches Wachstum eine bessere Haftung auf der Oberfläche zu erzeugen. Allerdings ist die beim Substrat bzw. zu beschichtenden Teil einzustellende Anfangstemperatur vom Material des zu beschichtenden Elementes abhängig und kann somit variieren bzw. vom obengenannten Wert abweichen. Die Anfangstemperatur kann in der Anlage, beispielsweise mittels einer elektronischen Infrarotheizung (6), gesteuert werden, wobei dies in der Regel in einem Bereich von ca. 80°C bis ca. 500°C möglich ist. Für die erfindungsgemässe Beschichtung wird der Inhalt des Rezipienten auf ca. 200°C - 250°C aufgeheizt.

Dann kann die Titanoxid-Schicht, die sich auf der Oberfläche von Titan und Titanlegierungen durch Oxidation automatisch gebildet hat, teilweise oder komplett entfernt werden, da sie die Anhaftung der Beschichtung reduzieren bzw. sogar verhindern kann. Durch das angelegte Vakuum im Rezipienten (5) wird dabei eine sofortige Zurückbildung der Oxid-Schicht verhindert und die Oberfläche ist somit perfekt vorbereitet für den anschliessenden Beschichtungsvorgang. Die beschriebene Vorbereitung der Oberfläche kann durch das Zünden einer Plasmareinigung bei sehr hoher Spannung (500 - 1400 V) im Vakuum geschehen. Dabei werden Metallionen mit sehr grosser Energie (dreifacher Schallgeschwindigkeit) auf die Oberfläche geschossen (lonenätzen). Dies geschieht mit einer solcher Wucht, dass die lonen nicht auf der Oberfläche haften bleiben. Zudem wird (wenig) Edelgas (z. B. Neon oder Argon) über ein Ventil (14) eingelassen, damit die abgesputterten lonen besser abgesaugt werden können. Eine Vorbereitung der Oberfläche ist jedoch auch über Trocken-Ätzen oder RIE-Verfahren (reactive ion etching) möglich.

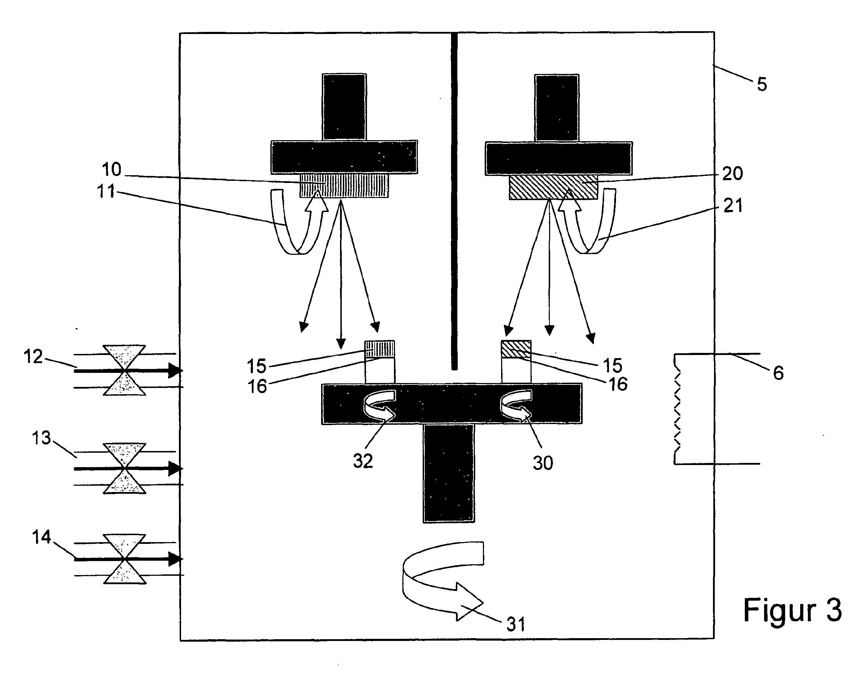

Anschliessend beginnt das eigentliche Beschichten (siehe auch Figur 3). Die erfindungsgemässe Beschichtung entsteht nun durch das abwechselnde Zünden verschiedener Metall-Targets (10 und 20). Besonders bevorzugt sind hierbei das abwechselnde Zünden eines Targets aus reinem Zirkon und eines Targets aus reinem Chrom, prinzipiell sind jedoch auch andere Metalle wie Gold, Silber, Eisen, Titan, Niob, Hafnium oder eine geeignete Legierung denkbar. Dadurch entsteht eine aufgedampfte Mehrschichten-Struktur (auch "Sandwich-Struktur" oder "layer by layer"). Das Verfahren wird mit mindestens zwei verschiedenen Metall-Targets durchgeführt, jedoch sind auch Verfahren geeignet, bei denen mehr als zwei Metalle oder Metalllegierungen abgeschieden werden. Besonders bevorzugt sind Beschichtungen aus zwei oder mehr Metall-Targets, bei denen der Anteil des ersten Metalls oder der ersten Metalllegierung im Vergleich zur Gesamtmenge an Metall in der Beschichtung zwischen 30 und 70 Atomprozent liegt, insbesondere zwischen 40 und 60 Atomprozent, besonders bevorzugt zwischen 45 und 55 Atomprozent liegt.

Auf einer Metallplatte (Target) (10), die z. B. aus reinstem Zirkon besteht, wird ein Lichtbogen (11) gezündet, der punktuell ca. 6.000°C hat. Das Target wird dabei von der Rückseite gekühlt, damit es nicht aufschmilzt, denn es sollte nie eine Temperatur von 250°C überschreiten. Die gelösten Metallionen werden nun mittels einer Bias-Spannung (Minus-Spannung) nochmals beschleunigt, damit sie sich auf der zu beschichtenden Oberfläche (14) "aufschweissen". Auf dem Flug vom Target zu dem zu beschichtenden Teil nimmt nun das Metall-Ion noch ein Atom Stickstoff auf (N₂), welches beim Beschichten im Rezipienten zur Verfügung gestellt wird (12). Analog wird ein zweiter Lichtbogen (21) auf einem zweiten Target (20), das z. B. aus reinstem Chrom besteht, gezündet. Durch die Montage der zu beschichtenden Elemente auf einem rotierenden Teller (30), entsteht die oben beschriebene Mehrschichtenstruktur. Um eine vollständige und gleichmässige Beschichtung zu erreichen, wird nicht nur der Teller rotiert (31), sondern auch die montierten Elemente an sich (32). In einer grossen Beschichtungsanlage sollte eine Dreifachrotation gewährleistet werden.

Ausgegangen wird von einem Anfangsvakuum von kleiner als 10⁻⁵ mbar, vorzugsweise von einem Vakuum von ca. 2,0 x 10⁻⁶ mbar. Bei der anschliessenden Beschichtung des Substrates wird das Vakuum durch Einlassen von Gasen wie Stickstoff (12) oder Azetylen (13) reduziert und pendelt sich in der Regel in einem Wertebereich von ca. 10⁻² bis 10⁻³ mbar ein. Dabei kann z. B. von reinem Stickstoff, von reinem Azetylen oder einem Gemisch von Stickstoff und Azetylen ausgegangen werden, wobei entsprechend Metallnitride, -carbide und/oder - carbonitride abgeschieden werden.

Gemäss einer bevorzugten Ausführungsvariante des erfindungsgemässen Verfahrens wird die Zusammensetzung des Gases während der Beschichtung verändert, so dass sich auch die Zusammensetzung in der Beschichtung während dem Beschichtungsvorgang ändert. Gemäss bevorzugter Ausführung werden anfänglich Nitride ausgeschieden. Zum Stickstoff wird nun Kohlenstoff in Form von Azetylen eingelassen, wodurch zunehmend Carbonitride in der Schicht mitabgeschieden werden. Der Kohlenstoffanteil verläuft vorzugsweise linear zunehmend, d. h. Azetylengas wird von einer anfänglichen Konzentration in der Grössenordnung von ca. 0 - 1 % auf ca. 30 - 35 % angehoben, womit entsprechend der Anteil Carbonitride in der Beschichtung zunimmt.

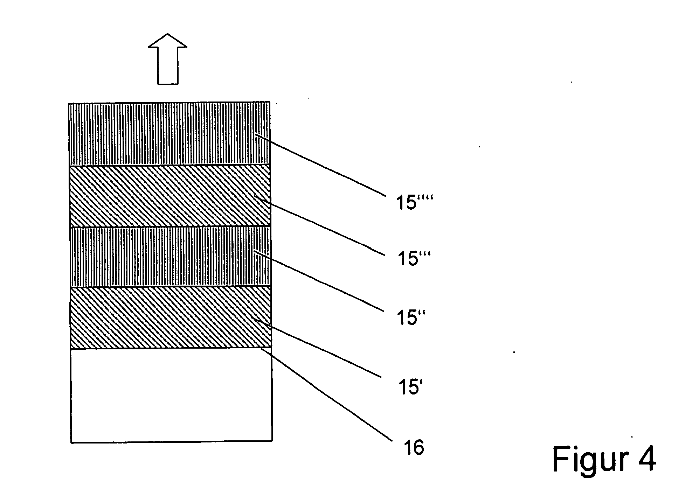

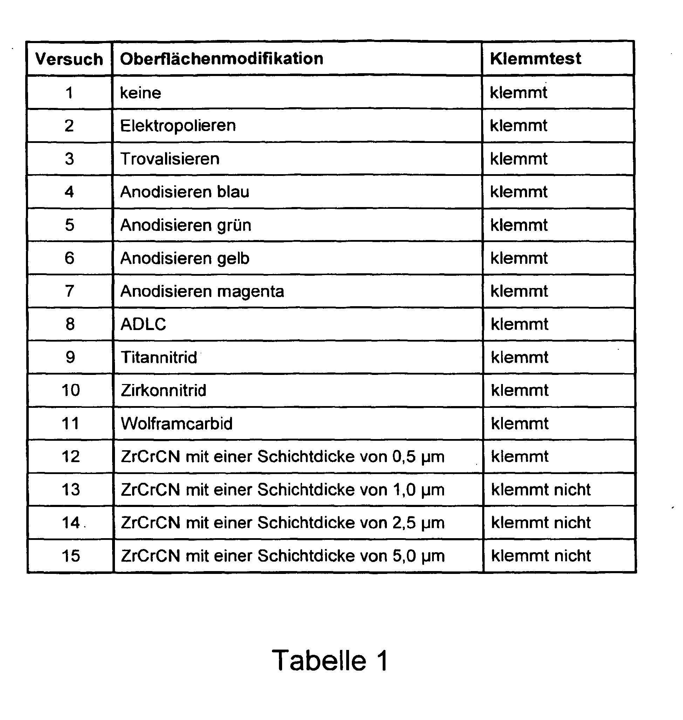

Dadurch, dass quasi ein atomarer Aufbau der Schicht vorliegt, muss mit einer "Aufwachsrate" der Beschichtung von ca. 0,5 µm pro Beschichtungsstunde gerechnet werden. Eine Beschichtung (15) dauert vom Schliessen der Tür des Rezipienten bis zum Wiederöffnen - je nach "Dicke" der Schicht - bis zu mehreren Stunden. In Figur 4 ist schematisch veranschaulicht, dass die erfinderische Beschichtung aus mehreren Einzelschichten (15', 15", 15"', 15"" usw.) besteht, insgesamt ergeben sich so bis zu 200 Einzelschichten aufgebracht auf die Oberfläche (16) des jeweiligen Elementes.

Eine bevorzugte Variante ist eine Multilayer-Beschichtung mit Zirkon und Chrom und Einleitung von Stickstoff und Azetylen (ZrCrCN). Übertragungsteile, die mit dieser Beschichtung überzogen wurden, verklemmen oder verschweissen sich nicht mehr mit den Implantaten, auch nicht bei der Lagerung in einer Flüssigkeit. In einem PVD-Verfahren wird dabei eine Kombination von Chrom und Zirkon unter Kohlenstoff- und Stickstoff-Partialdruck aufgedampft. Anfänglich wird dabei ZrN (Zirkonnitrid) alternierend mit CrN (Chromnitrid) aufgebracht. Im Verlauf des Verfahrens wird mittels Azetylenzugabe der Kohlenstoffgehalt von 0.5% auf bis zu 60% erhöht. Insgesamt werden bis zu 200 Schichten aufgebracht, welche eine totale Schichtdicke von einigen Mikrometern darstellen. Der Prozess findet im Hochvakuum statt und die Übertragungsteile werden zur homogenen Beschichtung einer dreifach Rotation ausgesetzt.

Nach der Beschichtung werden die Elemente dem Rezipienten (5) entnommen und einer abschliessenden Reinigung unterworfen. Diese Reinigung dient zum einen der Entfernung von kleinen Verschmutzungen, die z. B. beim Belüften des Rezipienten aufgewirbelt werden und sich auf den beschichteten Elementen ablagern können. Zum anderen kann auf diese Weise die Oberflächeneigenschaft der Beschichtung noch etwas verändert und verbessert werden, je nach Wahl des Reinigungsmittels und -verfahrens. Für den Fachmann ist es kein Problem ein geeignetes Standardverfahren auszuwählen. Es sollte jedoch darauf geachtet werden, dass das Reinigungsmittel biokompatibel ist, damit eventuell anhaftenden Reste keine Probleme erzeugen. Als besonders geeignet erscheint ein wasserlösliches Reinigungsmittel, das die Oberfläche hydrophil macht und schnell abtrocknet.

Die erfindungsgemässe Herstellung umfasst somit ein PVD-Verfahren mit folgenden Schritten:
- Vorreinigung ausserhalb des Vakuums
- Positionieren der Elemente im Rezipienten
- Anlegen eines Vakuum
- Vorbehandlung der Titan- oder Titanlegierungs-Oberfläche durch Edelgas-Sputtern
- Abwechselnde Abscheidung von Chrom und Zirkon in Stickstoff- und Azetylen-Atmosphäre bei konstanter Temperatur
- Lineare Veränderung des Verhältnisses von Stickstoff und Azetylen
- Belüftung
- Reinigung mit einem geeigneten Reinigungsbad

Durch das epitaktische Wachstum werden die Spannungen in der Beschichtung auf ein Minimum reduziert und es entsteht eine hochreine, dünne, äusserst dichte, verschleiss- und korrosionsbeständige Schutzschicht. Diese ist zudem sterilisierbar, auch unter extremen Sterilisierungsbedingungen, ohne dass eine Partikelablösung beobachtet werden kann. Die Beschichtung weißt eine Härte von 3.600 HV sowie einen Reibwert trocken gegen Stahl gemessen von 0,18 auf.

Die erfinderische Beschichtung weist eine sehr gute Schichtqualität auf. Aufgrund einer starken Bindung in der Übergangs- bzw. Interface-Zone ergibt sich eine gute Haftung zwischen Substrat und Beschichtung. Dies bildet die Vorraussetzung für eine gute und dauerhafte Adhäsion der Beschichtung. Zudem ist es wichtig, dass die Beschichtung geringe innere Spannungen aufweist, die der Bindung bzw. der Haftung entgegenwirken würden. Bei abgeschiedenen Schichten treten aus zwei Gründen innere Spannungen als Druck- oder Zugspannungen (mit Kräften parallel) zur Oberfläche auf. Zum einen entstehen thermische Spannungen durch unterschiedliche Ausdehnungskoeffizienten von beschichtetem Teil und Beschichtung bei unterschiedlichen Abscheide- und Betriebstemperaturen. Zum anderen entstehen intrinsische Spannungen durch strukturelle Unordnung der Fremd- und Schichtatome. Bei niedrig schmelzenden Metallen dominieren die thermischen Spannungen, bei hochschmelzenden Metallen dominieren hingegen die intrinsischen Spannungen. Eine hohe Temperatur bei der Abscheidung begünstigt den Platzwechsel durch Volumendiffusion und reduziert die intrinsischen Schichtspannungen. Intrinsische Spannungen können zudem durch die Wahl geeigneter Abscheideparameter (Restgasdruck, Temperatur usw.) minimiert werden. Innere Schichtspannungen können durch Verformung der Schicht abgebaut werden, dies kann jedoch zu Rissbildung (bei Zugspannung), plastisches Fliessen bei Druckspannung mit Hügelbildung sowie zu Abplatzen bzw. Delamination der Schicht führen. Die Schichtspannungen wirken den Bindungskräften somit entgegen und reduzieren die Adhäsion der Schicht. Um diese zu minimieren werden bei der erfindungsgemässen Beschichtung mehrere aufeinanderfolgende dünne Schichten von jeweils 10 bis 50 nm, alternierend aus verschiedenen Metallen oder Metalllegierungen abgeschieden. Besonders vorteilhaft ist es, wenn während dem PVD-Verfahren ein Gradient im Kohlenstoffgehalt gefahren wird. Damit ergeben sich graduelle Änderungen der Zusammensetzung und damit der physikalischen Eigenschaften ohne sprunghafte Änderungen, was zu einer weiteren Reduzierung der inneren Schichtspannungen führt.

Ein weiterer Vorteil der erfindungsgemässen Beschichtung ist ihre Biokompatibilität. Eine sehr gute Gewebe- und Blutverträglichkeit wurde nachgewiesen. Dadurch ist sie geeignet für das Aufbringen auf Elemente, die in der Medizinaltechnik angewendet werden.

Die Schichtdicke ist mit Hilfe des beschriebenen Verfahrens leicht einstellbar, entsprechend des Anwendungsbereiches. Bei einer Beschichtung eines Übertragungsteils, das auf ein Dentalimplantat montiert wird, empfiehlt sich eine Schichtdicke von mindestens 1 µm und von nicht mehr als 10 µm, besonders bevorzugt von nicht mehr als 5 µm.

Zu den Anforderungen an eine qualitativ hochwertige Schicht zählt zum einen die Einhaltung einer genau definierten Schichtdicke, denn die beiden Elemente sollen weiterhin passgenau herstellbar sein und exakt aufeinander montiert werden. Auch bestimmt die Dicke die Eigenschaften der Beschichtung bezüglich Geschlossenheit und Härte. Die Aufdampfrate kann leicht über einen Schwingquarz kontrolliert werden, der mitbeschichtet wird, zudem muss der Abtrag beim Sputtern mitberücksichtigt werden. Eine hohe Reinheit der Schicht wird gewährleistet durch die Reinheit des verdampften Targetmaterials, die vorbereitenden Reinigungsschritte sowie durch den Restgasdruck. Wichtig ist zudem eine gute Kantenabdeckung, um keine offenen Stellen für den Prozess der "Kaltverschweissung" zu haben. Auch diese Bedingung wird durch die erfindungsgemässe Beschichtung erfüllt, insbesondere dadurch dass es sich nicht um eine Beschichtung mit einer einzigen Schicht handelt, sondern um eine Beschichtung mit mehreren Einzellagen. Es ist daher darauf zu achten, dass die einzelnen Schichten eine gute Homogenität aufweisen.

Um die Funktionalität der erfindungsgemässen Beschichtung zu testen wurde ein sogenannter "Klemmtest" durchgeführt (siehe Tabelle 1). Dazu wurden Standard-Dentalimplantate (Straumann^{®} Standard Plus ø 4,1 mm) aus Titan (ISO 5832-2) verwendet, Übertragungsteile gefertigt aus einer Titan-Legierung Ti6Al7Nb (ISO 5832-11) wurden mit verschiedenen Beschichtungen versehen. Der Test wurde mit Implantaten durchgeführt, die in nassem Zustand (150 mM NaCl-Lösung) mit den entsprechenden Übertragungsteilen montiert wurden. Danach wurden die Implantate mit Hilfe des Übertragungsteils bis zu einem definierten Drehmoment in ein Knochenersatzmaterial (Canevasit HGW 2082, überschliffene Version) eingedreht. Getestet wurde dabei, ob sich das Übertragungsteil anschliessend ohne Probleme und Hilfsmittel (ausgenommen vorgegebenes Kontern mit Halteschlüssel, Adapter und Ratsche) entfernen lassen konnte. Sogar beim Eindrehen eines Implantates und provozierten Bruch des Übertragungsteils an der Sollbruchstelle bei ca. 110 Ncm musste der verbleibende Rest des Übertragungsteils gut entfernt werden können. 90% der Reste sollten ohne zusätzliche Werkzeuge entfernt werden können. Wenn bei 10% der Rest des Übertragungsteils mit einer Pinzette (oder kleiner Zange) mit geringer Kraft entfernt werden kann, galt dies noch als funktionstüchtig, da keine klinische Gefährdung des Patienten vorliegen würde.

Das Übertragungsteil in Versuch 1 wurde weder oberflächenmodifiziert noch beschichtet. In Versuch 2 wurde das Übertragungsteil elektropoliert, um im Mikrobereich eine glatte Oberfläche zu erhalten. Dabei handelt es sich um ein elektrochemisches Verfahren zum Polieren von Metallen. Das anodisch geschaltete Übertragungsteil wurde mit Gegenkathoden versehen und in einem geeigneten Elektrolyten einer Gleichspannung unterworfen. In Versuch 3 wurde die Oberfläche im Mikrobereich durch Trovalisieren verändert. Dazu wurden die Übertragungsteile in eine grosse Trommel mit speziellen Scheuerteilchen gegeben. Die Trommel wurde dann 24 Stunden gedreht, durch das Aneinanderreiben wurden zum einen Stanzgrate entfernt und Kanten verrundet und zum anderen die Oberfläche gehärtet. Bei der Anodisierung in den Versuchen 4 bis 6 ist die Oberfläche anodisiert (Verfahren zur Oberflächenbehandlung nach DIN 8580). Dabei wurde Aluminum als Anode geschaltet und dann mittels Gleichstrom in Säure elektrolysiert. Es wurden unterschiedliche Schichtdicken ausgetestet, die Anodisierungen haben je nach Dicke eine andere Farbe. Bei der ADLC-Beschichtung (Versuch 8) wurde in einem CVD-Verfahren eine reine Kohlenstoffschicht in Diamant-Koordination (sp3) aufgebracht. In Versuch 9 und 10 wurde ein PVD-Verfahren (analog dem oben beschriebenen) durchgeführt, allerdings nur mit einem Target - einmal mit reinem Zirkon, einmal mit reinem Titan, beide Male unter Zusatz von Stickstoff. Im Fall 11 wurde als Target Wolfram unter Zusatz von Azetylen getestet. In den Versuchen 12 bis 15 wurde das Übertragungsteil mit dem erfindungsgemässen Verfahren beschichtet, d. h. mit zwei verschiedenen Metallen und mit Einleitung von Azetylen und Stickstoff, hier also ZrCrCN, allerdings mit unterschiedlichen Schichtdicken zwischen 0,5 und 5,0 µm.

Die Ergebnisse des Klemmtestes sind in Tabelle 1 veranschaulicht. Dieser ist zu entnehmen, dass nur das erfindungsgemässe Beschichtungsverfahren mit zwei verschiedenen Metallen das Problem des Kaltverschweissens löst, dies allerdings erst ab einer Schichtdicke von 1,0 µm.

Die Erfindung wird nun beispielsweise anhand eines Ausführungsbeispieles näher erläutert.

Die nachfolgend angeführten Arbeits- bzw. Prozessbedingungen beziehen sich auf die beim Durchführen des Beispieles einer Beschichtung für ein Übertragungsteil unter Verwendung einer Anlage vom Typ Hauzer 1000-6.

Die zu beschichtenden Übertragungsteile werden gereinigt in den Rezipienten bzw. Reaktionsofen eingebracht. Vor Beginn des Beschichtungsprozesses wird der Reaktionsraum evakuiert auf ein Vakuum von 1 x 10⁻⁶ mbar und das Substrat aufgeheizt auf eine Temperatur von ca. 270 °C. Vor Beginn der Beschichtung wird die Oberfläche des zu beschichtende Übertragungsteil zunächst mittels lonenätzen gereinigt werden. Dabei werden folgende Prozessparameter eingestellt:

| | |
|---|---|
| Bias: | 500 - 1400 Volt |
| Verdampfer: | Einzeln bei 60 - 110 Ah |
| Laufzeit: | 8 s |

Während der Dauer des Reinigungsprozesses läuft eine Turbomolekularpumpe auf voller Leistung, um allfällig beim Reinigen entstehende Verunreinigungen sofort aus dem Bearbeitungsraum entfernen zu können. Zusätzlich wird der Reinigungsprozess durch die Zugabe kleinster Mengen von Argon unterstützt.

Bei Beginn der Beschichtungsphase wird ein Bias am Substrat angelegt von anfänglich ca. 45 V, welches im Verlaufe der Beschichtungsphase bei steigendem Azetylenanteil auf einen Bereich von ca. 25 - 40 V reduziert wird. Der Verdampfer wird bei der Beschichtungsphase gepulst oder kontinuierlich betrieben, wobei bei fortschreitender Dauer der Beschichtungsphase die Stromstärke bei steigender Azetylenkonzentration reduziert wird.

Während der Beschichtungsphase wird ein weitgehend konstanter Stickstofffluss in der Grössenordnung von ca. 40 ppm gewählt, während gleichzeitig der Azetylenfluss von ca. 0,5 - ca. 35 % steigend gewählt wird, was ca. 1 bis 25 - 30 ppm ergibt, wobei der Anstieg des Azetylenflusses vorzugsweise linear ansteigend eingestellt wird. Der Druck im Rezipienten bzw. Reaktionsraum sollte im Bereich von ca. 5 x 10⁻³ ― 1 x 10⁻² mbar sein, und während dem gesamten Beschichtungsprozess läuft die obenerwähnte Turbomolekularpumpe lediglich mit einer Leistung von ca. 60 - 80 %. Nach dem Beschichten wird der Reaktionsraum vorsichtig belüftet und die beschichteten Übertragungsteile in einem Standardverfahren abschliessend gereinigt.

Desweiteren ist zu beachten, dass Elemente aus Zirkon oder Zirkon-Legierungen analog wie Elemente - aus Titan oder Titan-Legierungen den Effekt des Kaltverschweissens aufweisen. Die erfindungsgemässe Beschichtung ist daher nicht auf Elemente aus Titan oder Titan-Legierungen beschränkt, sondern kann analog für Elemente aus Zirkon oder Zirkon-Legierungen angewendet werden, um die vorbeschriebene Aufgabe zu lösen. Damit sind in dieser Erfindung Verfahren, Elemente, Beschichtungen und Kombinationen von Elementen mit eingeschlossen, bei denen die Elemente statt aus Titan oder Titan-Legierung aus Zirkon oder Zirkon-Legierung bestehen. Die vorangegangene Offenbarung gilt analog für diese Verfahren, Elemente, Beschichtungen und Kombinationen von Elementen.

## Patentansprüche

1. PVD-Verfahren zur Beschichtung von Elementen aus Titan oder Titan-Legierung bzw. Zirkon oder Zirkon-Legierung, aufweisend mindestens folgende Schritte
a) Positionieren der Elemente in einem Rezipienten;
b) Anlegen eines Vakuum;
c) Abscheidung eines ersten Metalls in Stickstoff- und Azetylen-Atmosphäre;
d) Abscheidung eines zweiten Metalls in Stickstoff und Azetylen-Atmosphäre;
e) Wiederholung der Schritte c) und d) bis eine Schichtdicke von mindestens 1 µm erreicht ist;
f) Belüftung des Rezipienten;
g) Entnahme der Elemente aus dem Rezipienten;
h) Reinigung der Elemente mit einem geeigneten Reinigungsbad,
**dadurch gekennzeichnet, dass**
das erste Metall Chrom und das zweite Metall Zirkon ist.

2. Verfahren nach Anspruch 1, **gekennzeichnet dadurch dass** nach dem Schritt b) eine Vorbehandlung der Oberfläche durchgeführt wird, insbesondere durch Sputtern mit Edelgasen.

3. Verfahren nach Anspruch 1 oder 2, **gekennzeichnet dadurch dass** während Schritt e) das Verhältnis von Stickstoff und / oder Azetylen verändert wird.

4. Verfahren nach Anspruch 3, **gekennzeichnet dadurch dass** die Veränderung linear ist.

5. Verfahren nach Anspruch 1 bis 4, **gekennzeichnet dadurch dass** mehr als zwei Metalle oder Metalllegierungen abgeschieden werden.

6. Element aus Titan oder Titan-Legierung mit einer Beschichtung hergestellt in einem Verfahren nach Ansprüchen 1 bis 5.

7. Element nach Anspruch 6, bei dem es sich um ein Implantat, ein Dentalimplantat, eine Knochenschraube oder ein Übertragungsteil handelt

8. Beschichtung auf einem Element aus Titan oder Titan-Legierung hergestellt in einem Verfahren nach Ansprüchen 1 bis 5.

9. Beschichtung nach Anspruch 8, **gekennzeichnet dadurch dass** der Anteil des ersten Metalls im Vergleich zur Gesamtmenge an Metall in der Beschichtung zwischen 30 und 70 Atomprozent liegt, insbesondere zwischen 40 und 60 Atomprozent, besonders bevorzugt zwischen 45 und 55 Atomprozent liegt.

10. Kombination von Elementen, bei denen mindestens ein Element zumindest teilweise mit einer Beschichtung nach Ansprüchen 8 oder 9 versehen ist.

## Claims

1. PVD method for the coating of elements made from titanium or titanium alloy, or zirconium or zirconium alloy, respectively; comprising at least the following steps:
a) positioning the elements in a recipient,
b) creation of a vacuum,
c) deposition of a first metal in a nitrogen and acetylene atmosphere,
d) deposition of a second metal in a nitrogen and acetylene atmosphere,
e) repetition of steps c) and d) until a layer thickness of at least 1 µm has been achieved,
f) ventilation of the recipient,
g) removal of the elements from the recipient,
h) cleaning of the elements with a suitable clearing bath,
**characterized in that**
the first metal is chromium and the second metal is zirconium.

2. Method according to Claim 1, **characterized in that** after step b), a pretreatment of the surface is carried through, in particular by sputtering with noble gases.

3. Method according to Claim 1 or 2, **characterized in that** during step e), the ratio of nitrogen and/or acetylene is changed.

4. Method according to Claim 3, **characterized in that** the change is linear.

5. Method according to Claims 1 through 4, **characterized in that** more than two metals or metal alloys are deposited.

6. Element made from titanium or titanium alloy with a coating, manufactured in a method according to Claims 1 through 5.

7. Element according to Claim 6 which is an implant, a dental implant, a bone screw or a transmission piece.

8. Coating on an element made from titanium or titanium alloy, manufactured in a method according to Claims 1 through 5.

9. Coating according to Claim 8, **characterized in that** the ratio of the first metal to the total amount of metal in the coating is between 30 and 70 atomic percent, in particular between 40 and 60 atomic percent, particularly preferably between 45 and 55 atomic percent.

10. Combination of elements where at least one element is at least partially provided with a coating according to Claim 8 or 9.

## Revendications

1. Procédé de couchage par métal dur (PVD - physical vapour deposition), pour le revêtement d'éléments en titane ou en alliage de titane ou en zircon ou en alliage de zircon, présentant au moins les étapes suivantes :
a) positionnement des éléments dans un récipient ;
b) application d'un vide ;
c) déposition d'un premier métal dans une atmosphère d'azote et d'acétylène ;
d) déposition d'un deuxième métal dans une atmosphère d'azote et d'acétylène ;
e) répétition des étapes c) et d), jusqu'à obtention d'une épaisseur de couche d'au moins 1 µm ;
f) aération du récipient ;
g) prélèvement des éléments hors du récipient ;
h) nettoyage des éléments avec un bain de nettoyage approprié ;
**caractérisé en ce que**
le premier métal est le chrome et le deuxième métal est le zircon.

2. Procédé selon la revendication 1, **caractérisé en ce que**, après l'étape b), est effectué un prétraitement de surface, en particulier un dépôt par pulvérisation avec des gaz rares.

3. Procédé selon la revendication 1 ou 2, **caractérisé en ce que**, pendant l'étape e), le rapport entre azote et/ou acétylène est modifié.

4. Procédé selon la revendication 3, **caractérisé en ce que** la modification est linéaire.

5. Procédé selon les revendications 1 à 4, **caractérisé en ce que** la déposition implique plus de deux métaux ou alliages métalliques.

6. Elément en titane ou en alliage de titane, avec un revêtement fabriqué suivant un procédé selon les revendications 1 à 5.

7. Elément selon la revendication 3, pour lequel il s'agit d'un implant, d'un implant dentaire, d'une vis à os ou d'une pièce de transmission.

8. Revêtement sur un élément en titane ou en alliage de titane, fabriqué suivant un procédé selon les revendications 1 à 5.

9. Revêtement selon la revendication 8, **caractérisé en ce que** la proportion du premier métal par rapport à la quantité totale de métal dans le revêtement est située entre 30 et 70 pourcents atomiques, en particulier entre 40 et 60 pourcents atomiques, de manière particulièrement préférée entre 45 et 55 pourcents atomiques.

10. Combinaisons d'éléments, pour lesquels au moins un élément est muni, au moins partiellement, d'un revêtement selon la revendication 8 ou 9.
